Europäisches Patentamt

European Patent Office

Office européen des brevets

(11) Veröffentlichungsnummer: **0 265 851 B1**

## EUROPÄISCHE PATENTSCHRIFT

(45) Veröffentlichungstag der Patentschrift: **16.02.94**

(21) Anmeldenummer: **87115527.1**

(22) Anmeldetag: **23.10.87**

(51) Int. Cl.5: **G01N 33/569**, G01N 33/577, G01N 33/543, //G01N33/545

(54) **Verfahren zur Bestimmung von Anti-HIV, Mittel dazu und ihre Verwendung in diesem Verfahren.**

(30) Priorität: **27.10.86 DE 3636540**

(43) Veröffentlichungstag der Anmeldung:
**04.05.88 Patentblatt 88/18**

(45) Bekanntmachung des Hinweises auf die Patenterteilung:
**16.02.94 Patentblatt 94/07**

(84) Benannte Vertragsstaaten:
**AT BE CH DE ES FR GB IT LI LU NL SE**

(56) Entgegenhaltungen:
EP-A- 0 136 798
EP-A- 0 181 150
EP-A- 0 226 903
WO-A-85/04903
WO-A-86/04336

JOURNAL OF VIROLOGICAL METHODS, Band 12, 1985, Elsevier Science Publishers B.V. (Biomedical Division), Amsterdam (NL); A.R. NEURATH et al., pp. 85-92&NUM;

JOURNAL OF VIROLOGICAL METHODS, Band 11, 1985; .R. NEURATH et al., pp. 75-82&NUM;

(73) Patentinhaber: **BEHRINGWERKE Aktiengesell-schaft**
**Postfach 1140**
**D-35001 Marburg(DE)**

(72) Erfinder: **Krupka, Udo, Dr.**
**Oberer Eichweg 29**
**D-3550 Marburg(DE)**
Erfinder: **Buck, Thomas, Dr.**
**Brunnenquell 14**
**D-3551 Lahntal(DE)**
Erfinder: **Pauly, Hans Erwin, Dr.**
**Finkenstr. 1**
**D-3563 Dautphetal(DE)**

**Beschreibung**

Die Erfindung betrifft ein Verfahren zur immunchemischen Bestimmung von HIV-spezifischen Antikörpern, welches das Kompetitionsprinzip verwendet, sowie für dieses Verfahren geeignete Mittel und ihre Verwendung. Das Verfahren ist zum hochsensitiven und spezifischen Nachweis und der Bestimmung von HIV-spezifischen Antikörpern der IgG- und IgM-Klasse beim Menschen geeignet.

Als Erreger der kürzlich erkannten infektiösen Erkrankung AIDS (Erworbenes Immundefekt-Syndrom), die eine hohe Sterblichkeitsrate aufweist, wird ein Virus (Retrovirus) bzw. eine eng verwandte Gruppe von Viren angesehen, die neuerdings als Human Immune Deficiency Virus (HIV) bezeichnet werden, aber auch T-Lymphotropic Virus Type III (HTLV III) oder Lymphadenopathy Associated Virus (LAV) genannt werden.

Das Virus kann durch Human-Blut und kontamierte Blutpräparate übertragen werden und konnte aus Liquor, Samenflüssigkeit, Tränenflüssigkeit und Speichel isoliert werden.

Die serologische Bestimmung von HIV-spezifischen Antikörpern wird vorgenommen, um zu klären, ob ein Individium mit dem Virus Kontakt hatte und als potentieller Träger des infektiösen Agens anzusehen ist. Eine wesentliche Bedeutung der Antikörper-Bestimmung besteht neben der Anwendung als Diagnose-Instrumentarium für AIDS (zusammen mit anderen Untersuchungen und der Anamnese) in der Verringerung des Risikos einer Übertragung des Krankheitserregers via Transfusion, Organ-Transplantation, Blutpräparate oder Samen durch Ausschluß der Spenden von Antikörper-positiven Personen. Darüberhinaus ist die HIV-Antikörper-Bestimmung von Wichtigkeit bei Angehörigen sogenannter Risikogruppen (Homosexuelle, Drogenabhängige, Hämophile, Kinder infizierter Mütter) ebenso wie bei epidemiologischen Untersuchungen.

Der Nachweis von Anti-HIV unter Verwendung der Western Blot-Technik wie er beispielsweise in USP 4,520,113 beschrieben ist, gilt als der zur Zeit empfindlichste Nachweis von HIV-spezifischen Antikörpern. Dabei wird angereichertes und inaktiviertes HIV-Proteinmaterial mittels Elektrophorese nach Molekulargewichten getrennt und anschließend auf Nitrozellulosepapier übertragen, bevorzugt elektrophoretisch. Mit derart immobilisiertem Antigen kann in Abänderung zur USP 4,520,113, wo ein RIA verwendet wird, ohne Einbußen an Empfindlichkeit ein indirekter ELISA durchgeführt werden, wobei durch Verwendung schwerlöslicher Farbstoff-Systeme bei der Enzymreaktion es möglich ist, die Banden, an denen eine Immunreaktion stattgefunden hat, zu visualisieren. Es können dann nach Eichung mit entsprechendem Kontrollmaterial angefärbte Virus-spezifische Banden von Nicht-Virusantigen-spezifischen sowie kreuz- oder unspezifisch-reagierenden Kontaminationen unterscheiden, und somit eine Diskriminierung zwischen Echt- und Falsch-Positivreaktionen vollzogen werden.

Weiterhin ist ein Verfahren beschrieben, das ein kompetitiver ELISA ist und hinsichtlich Spezifität und Empfindlichkeit die Kriterien der Western Blot Technik angeblich erfüllt (Ärzte-Zeitung/Nr.1, 25./26.10.85, S. 20).

In diesem Verfahren werden unverdünnte Proben benutzt. Mit ihm sind HIV-spezifische Antikörper der IgG- und IgM-Klasse gleichzeitig nachweisbar. Das Verfahren kann mit einem Testkit der Firma Wellcome, bezeichnet als Wellcozyme Anti-HTLV III, durchgeführt werden. Für die Festphase werden Microtestplatten verwendet, an die gereinigte HIV-Antikörper gebunden sind und an diese wiederum HIV-Antigene immunchemisch immobilisiert sind. Die Proben werden in den Cavitäten (Wells) zusammen mit Meerrettich-Peroxidase-markiertem Anti-HIV inkubiert. Dabei konkurrieren die Antikörper gegen HIV aus der Probe mit dem markierten Anti-HIV um die Bindungsstellen des HIV an der Festphase. Die Menge in den Wells gebundenen markierten Antikörpers ist umgekehrt proportional zur Anti-HIV-Konzentration in der Probe.

Sehr häufig können für Falsch-Positiv-Resultate bei anderen derzeitig verwendeten Tests humane Leukozyten-Antigene Klasse II (HLA II, insbesondere DR4) verantwortlich gemacht werden, die von den zur HIV-Züchtung üblicherweise verwendeten humanen Lymphozyten-Zellinien stammen, und die insbesondere bei polytransfundierten Patienten (Anti-HLA DR4) zu Falsch-Positiven-Resultaten führen können.

Diese Art von "unspezifischen" Reaktionen wird bei dem erwähnten kompetitiven Testkit zum Nachweis HIV-spezifischer Antikörper (Walgate R., Beardsley T., (1986) Nature 320, 96) durch die Verwendung der humanen Zellinie CCRF-CEM zur Viruszucht ausgeschlossen, da diese Zellen keine HLA der Klasse II tragen.

Wurde bei dem beschriebenen kompetitiven ELISA HIV verwandt, das in der für bessere Ausbeute bekannten H9-Zelllinie gezüchtet worden war, so war festzustellen, daß extrem große Mengen von Virus-Ausgangsmaterial erforderlich sind, um einerseits die Fängerantikörper für die Immobilisierung auf der Festphase zu reinigen und andererseits die für eine maximale Sensitivität erforderliche Sättigung der auf den Kunststoff immobilisierten Fängerantikörper mit Antigen zu erzielen, daß weiterhin die immunadsorptive Reinigung von humanem Serum im Hinblick auf eine Verbesserung der Spezifität nur unbefriedigend verläuft, wenn auf H9 gezüchtetes und damit mit nukleären, mitochondrialen und leukozytären Antigenen kontaminiertes HIV-Antigenmaterial verwendet wird. Ferner wurden nur sehr geringe Ausbeuten spezifischer humaner Antikörper erhalte, was die Anwendung als Screeningtest aus ökonomischen Gründen unmöglich macht.

In der nachveröffentlichten Patentanmeldung EP 0 226 903 wird eine Methode zum Nachweis von Antikörpern gegen HIV beschrieben, bei der zwei Nachweissysteme zum Nachweis von HIV Antikörpern, die an immobilisierte, rekombinante p24, bzw. p41 Peptide binden, verwendet werden.

Es wurde gefunden, daß ein kompetitives Verfahren zum Nachweis und zur Bestimmung von Antikörpern gegen HIV sowohl der IgM- wie der IgG-Klasse möglich ist, bei Verwendung einer Festphase, an die HIV-Antigene, die auch mit aus der Wirtszelle stammenden Antigenen verunreinigt sein können, direkt, also ohne Antikörper vermittelte Bindung, gebunden sind, wenn markierte Antikörper gegen HIV verwendet werden, die weder mit Antigenen der Wirtszelle noch mit irgendwelchen Antigenen aus humanem Gewebe reagieren.

Gegenstand der Erfindung ist somit ein immunchemisches, kompetitives Verfahren zum Nachweis und zur Bestimmung von Antikörpern gegen HIV unter Verwendung einer Festphase bestehend aus einem Träger und daran gebundenen HIV Virus Proteinen erhalten aus HIV Kulturen und einer markierten Antikörperpräparation, wobei die markierten und unmarkierten Antikörper um die Bindungsstelle an der Festphase konkurrieren und die Festphasenproteine direkt oder über einen nicht-immunchemisch bindenden Spacer an den Träger gebunden sind nach einem der Ansprüche 1 - 5.

Gegenstand der Erfindung ist auch ein Testbesteck zum Nachweis und zur Bestimmung von Antikörpern gegen HIV in einem immunchemischen Verfahren nach einem der Ansprüche 6 - 9.

Gegenstand der Erfindung ist ferner die Verwendung eines Testbesteckes nach den Ansprüchen 6 - 9 in einem Verfahren zum Nachweis und zur Bestimmung von Antikörpern gegen HIV.

Es ist vorteilhaft, daß die Sensitivität des erfindungsgemäßen Verfahrens der Empfindlichkeit der Western Blot-Technik entspricht, da selbst Proben mit niedrigen HIV-Antikörper-Titern zu einer signifikanten Inhibition der Bindung der markierten Antikörper führen, was z. B. bei Verwendung eines Enzymmarkers eine visuelle Bewertung der Farbstoffentwicklung erlaubt und eine Diskriminierng von positiven und negativen Proben mit außerordentlich hoher Trennschärfe ermöglicht, daß auch frühe Serokonversionen, durch HIV-spezifische IgM-Antikörper verursacht, sicher erfaßt werden, daß keine Interferenzen und damit Falsch-Positiv-Resultate durch humane Leukozyten-Antigene (HLA), nukleäre Antigene oder mitochondriale Antigene auftreten, so daß eine Prüfung, ob ein Positiv-Ergebnis spezifisch ist, mit Hilfe von herkömmlichen Kontrollantigenen aus der Wirtszelle überflüssig ist, und daß in Seren, Citrat-, Heparin- und EDTA-Plasmen sowie in recalcifizierten Plasmen humanen Ursprungs eine sichere Bestimmung von HIV-spezifischen Antikörpern gewährleistet ist und eine Inaktivierung der Proben über 30 min bei 55 °C (HIV-Inaktivierung) und selbst über 10 h bei 60 °C (Hepatitis B-Virus-Inaktivierung) zu keinen Falsch-Positiven-Resultaten führt.

Für die Festphase geeignete Antigenpräparationen von HIV können hergestellt werden, indem zunächst HIV-positive permanente T-Zellen gewonnen werden wie in WO85/04897 beschrieben und eine HIV-Antigenpräparation gewonnen wird wie in USP 4,520,ll3 dargestellt. Dabei wird zunächst das HIV nach Transmission auf die humane T-Zellinie H9 durch Cokultivierung mit Lymphozyten eines AIDS-Patienten propagiert und anschließend durch Ultra-Zentrifugation vom Wirtszell-Kulturüberstand abgetrennt. Nach Entfernen von Zelltrimmern und Fett wird das Virusmaterial über eine Sucrose-Dichtegratienten-Zentrifugation gereinigt und abschließend durch Zugabe von [R]Triton und Hitzebehandlung inaktiviert.

Als Trägermaterial für die Festphase sind Kunststoffe wie Polystyrol, Polyvinylchlorid, Polyamid und andere synthetische Polymere, natürliche Polymere wie Zellulose, sowie derivatisierte natürliche Polymere wie Zelluloseacetat und Nitrozellulose als auch Glas, insbesondere als Glasfaser geeignet.

Die Träger können die Form von Kugeln, Stäben, Röhrchen, und Microtestplatten haben. Flächenförmige Gebilde wie Streifenpapiere, Plättchen und Membranen sind ebenfalls geeignet. Die Oberfläche der Träger kann sowohl für wässrige Lösungen durchlässig als auch undurchlässig sein.

Bevorzugte Träger sind Kugeln, Röhrchen, Streifenpapiere und Membranen. Besonders bevorzugte Träger sind Microtestplatten.

Die Festphase wird hergestellt, indem eine Antigenpräparation von HIV irreversibel an den Träger gebunden wird.

Eine irreversible Bindung im Sinne der Erfindung liegt beispielsweise vor bei

1) einer adsorptiven Bindung, die nicht durch immunchemische Mittel, wie hochaffine Antikörper und auch nicht durch die in dem Verfahren verwendeten Mittel, wie markierte Antikörper, Verdünnungs- und Pufferlösungen gespalten wird,

2) einer über einen nicht immunchemisch bindenden Spacer vermittelte, bioaffine Bindung, wobei der Spacer aus Biotin und Avidin oder aus anderen Paarungen von Rezeptoren und Liganden bestehen kann,

3) einer kovalenten direkten Bindung

4) einer kovalenten, durch einen chemisch bifunktionellen Spacer vermittelten Bindung

Bevorzugt wird die kovalente Bindung im Falle der Verwendung von wasserdurchlässigen Trägern, sowie die adsorptive Bindung im Falle der Verwendung von wasserdurchlässigen als auch wasserundurchlässigen Trägern.

Besonders bevorzugt wird die direkte adsorptive Bindung von HIV Antigenpräparationen an mit Gammastrahlen behandeltes Polystyrol als Träger.

Für die Markierung vorgesehene Antikörper werden ausgewählt auf Grund der Ergebnisse der Western Blot-Analyse von Seren von AIDS-Patienten.

Der Nachweis von weiteren Antikörperspezifitäten, nämlich solchen für die als p18, p30, p55 bezeichneten Core-Proteine und die als p53 und p65 bezeichnete HIV Polymerasen bedeutet eine zusätzliche Absicherung des Nachweises von Anti-HIV-positiven Proben.

Um eine falsch positive Diagnose auszuschließen, dürfen die für die Markierung vorgesehenen Antikörper keine Spezifität haben, die Bestandteile humanen Gewebes erkennen, wie beispielsweise Leukozytenantigene, nukleäre und mitochondialen Proteine.

Für die Markierung können Antikörper von AIDS-Patienten verwendet werden, wenn sie die nach der Western Blot-Technik ermittelten, zuvor beschriebenen Kriterien erfüllen.

Weiterhin können polyklonale Antikörper, gewonnen aus Seren von mit HIV immunisierten Tieren, verwendet werden, wenn diese nach Adsorption der Spezifitäten, die Bestandteile der Wirtszelle und des Kulturmediums erkennen, nach Prüfung im Western Blot die genannten Kriterien erfüllen.

Monoklonale Antikörper sind ebenfalls geeignet. Es müssen dann mindestens zwei verwendet werden in einer der genannten Kombinationen von Spezifitäten, also beispielsweise in der Kombination Anti-p24 und Anti-gp41.

Als Marker können verwendet werden radioaktive Isotope, fluoreszierende und chemilumineszierende Farbstoffe sowie Enzyme, deren Nachweis durch chromogene, luminogene oder fluorogene Substratsysteme möglich ist.

Die Markierung erfolgt nach Verfahren, die für die genannten Marker als Stand der Technik beschrieben sind.

Im Falle der Markierung der Antikörper mit Peroxidase kann die Periodat Technik nach Nakane et al., 1974, J. Histochem. Cytochem. 22, 1084-1090, angewandt werden oder ein Verfahren gemäß Ishikawa et al., 1983, J. Immunoassay 4, 209-327, in dem die Partner mit einem heterobifunktionellen Reagenz verknüpft werden.

Das erfindungsgemäße Verfahren kann beispielsweise durchgeführt werden, indem in die Cavitäten (Wells) einer Microtestplatte, die eine zuvor beschriebene HIV-Antigenpräparation gebunden enthält,

a) gleichzeitig die Probe und eine Lösung des markierten Antikörpers gegebenenfalls zuerst die Probe und nach einer gewissen Zeit eine Lösung des markierten Antikörpers gegeben, nach einer bestimmten Zeit der Inkubation die Lösung entfernt und die Wells mit einem Puffer gewaschen werden und anschließend die Markierung in den Wells gemessen wird oder

b) zuerst eine Lösung enthaltend den markierten Antikörper gegeben wird, nach einer bestimmten Zeit der Inkubation die Lösung entfernt und die Wells mit einem Puffer gewaschen, die Microtestplatte gegebenenfalls getrocknet, anschließend die verdünnte Probe zugegeben und eine bestimmte Zeit inkubiert, dann die Probe aus den Wells entfernt und die Markierung in der Probe gemessen wird.

Das erfindungsgemäße Verfahren kann auch durchgeführt werden bei Verwendung eines diagnostischen Elements, das die Festphase und in trockener Form einen Teil oder auch alle erforderlichen Reagenzien enthält.

Das folgende Beispiel stellt eine Ausführungsform der Erfindung dar, ohne sie jedoch darauf zu beschränken.

Beispiel

1. Bindung von HIV-Antigen an Microtestplatten

Eine gemäß USP 4,520,113 hergestellte, über Sucrose-Dichtegradienten-Zentrifugation gereinigte und hitzeinaktivierte Antigenpräparation von HIV wurde mit 100 mmol/l Natriumbicarbonat, pH 9.6 auf 50 µg/ml Protein vorverdünnt. Von dieser Verdünnung wurde eine 2-fach Verdünnungsreihe in dem gleichen Puffer mit 10 Verdünnungen angelegt, also eine Reihe mit den Konzentrationen 50, 25, 12.5, 6.25, 3.12, 1.56, 0.78, 0.39, 0.2 und 0,1 µg/ml erhalten. 100 µl einer jeden Verdünnung wurde in jeweils 16 Wells von Microtestplatten, Typ B der Firma Nunc, Roskilde, Dänemark gegeben. Die mit den Verdünnungen gefüllten Testplatten wurden 18 Stunden bei 20°C belassen, dann wurden die Lösungen in den Wells abgesaugt und die Wells 3-4 mal mit 200µl einer Lösung von 10 g/l Rinderserumalbumin in phosphatgepufferter physiologischer Kochsalzlösung, pH 7.4 (PBS) durch Füllen und Absaugen gewaschen und die Testplatten anschließend über Kieselgel bei 20 °C getrocknet.

2. Herstellung eines Peroxidase-markierten Antikörpers gegen HIV

Ein nach der Western Blot-Technik ausgewähltes Serum eines AIDS-Patienten welches Antikörperspezifitäten, für die Core-Proteine p18, p24, p30 und p55, für die Hüll-Proteine gp41 und gp120 sowie für die HIV Polymerasen p53 und p65 enthielt, wurde durch Chromatographie an DEAE-Zellulose fraktioniert. Dazu wurde das Serum gegen Puffer von 30 mmol/l $Na_2HPO_4/NaH_2PO_4$, pH 7.0 dialysiert und auf eine Säule, gefüllt mit DEAE Cellulose DE 32 (Whatman) und equilibriert mit dem gleichen Puffer, gegeben. Die im Durchlauf erhaltene IgG-Fraktion wurde gegen PBS dialysiert und auf 4 mg/ml Protein eingestellt.

Eine weitere Prüfung in der Western Blot-Technik ergab, daß alle oben angeführten Antikörperspezifitäten in der IgG-Fraktion wiedergefunden wurden.

Die IgG-Fraktion wurde anschließend mit N-gamma-Maleimidobutylyloxisuccinimid (GMBS), bezogen von der Fa. Behring Diagnostics, umgesetzt wie von Tanamori et al., 1983 in J. Immunol. Meth. 62, 123-131 beschrieben. 2-Iminothiolanhydrochlorid (Fa. Sigma, Kat. Nr. I 6256) wurde mit Meerrettich-Peroxidase (POD), bezogen von der Fa. Boehringer Mannheim, Kat. Nr. 413470, umgesetzt wie von King et al. 1978 in Biochem. 17, 1499-1506 beschrieben. Aus dem GMBS-IgG Konjugat und dem Iminothiolan-POD Konjugat wurde ein IgG-POD Konjugat hergestellt wie von Tanamori beschrieben.

Die erhaltene Lösung des IgG-POD Konjugats hatte einen Proteingehalt von 440 $\mu$g/ml. Das Verhältnis von POD zu IgG wurde mit 2.5 bestimmt. Die Lösung wurde anschliessend auf 500 ng/ml IgG-POD mit einer Lösung von 50 ml/l foetalem Kälberserum, 5 g/l Polyoxiethylen-(20)-sorbitan Monolaureat ([R]Tween 20) in PBS verdünnt und erhielt die Bezeichnung Anti-HIV-POD.

3. Herstellung einer TMB-Substratzubereitung

Zum Nachweis von Anti-HIV-POD wurde ein Substratsystem oder eine Substratzubereitung verwandt, enthaltend Wasserstoffperoxid und Tetramethylbenzidin (TMB), welche aus zwei Stammlösungen hergestellt wurde.

Stammlösung 1: TMB-Dihydrochlorid wurde unter Rühren in einer Konzentration von 5 g/l, d. h. von 16 mmol/l in bidestilliertem Wasser gelöst und mit 5 normaler Salzsäure auf pH 1.5 eingestellt. Zu dieser Lösung wurde Penicillin G unter Rühren in einer Endkonzentration von 200 mg/l, d. h. von 0.56 mmol/l zugesetzt.

Stammlösung 2: zu 900 ml bidestilliertem Wasser wurde 1.4 ml Eisessig, 1.5 ml 1 normale NaOH und 250 mg, d. h. 3 mmol $H_2O_2$ als Harnstoff-Wasserstoffperoxid-Addukt zugesetzt. Nach vollständigem Lösen wurde mit bidestilliertem Wasser auf 1 l aufgefüllt.

TMB-Substratzubereitung: Ein Volumenteil der Stammlösung 1 und 10 Volumenteile der Stammlösung 2 wurden miteinander vermischt.

4. Optimierung des Verfahrens

16 Seren, die nach der Western Blot-Technik als HIV negativ bestätigt worden waren, wurden mit Anit-HIV-POD 1:5 verdünnt (1 Volumenteil Serum plus 4 Volumenteile Anti-HIV-POD).

In alle Wells der mit HIV-Antigenpräparation behandelten Microtestplatten wurden jeweils 125 $\mu$l der Verdünnung gegeben und 1 h bei 27 °C inkubiert. Die Verdünnungen wurden abgesaugt und die Wells viermal mit einer Lösung von 1 g/l [R]Tween 20 in PBS durch Füllen und Absaugen gewaschen. Anschließend wurde in jede Well 100 $\mu$l TMB-Substratzubereitung gegeben und 30 min bei 20 bis 22 °C inkubiert. Die Inkubation wurde beendet durch Zugabe von jeweils 100 $\mu$l 1 normaler Schwefelsäure. Die Extinktion der Lösungen wurde gegen PBS bei 450 nm gemessen.

Die Lösungen der Wells, die mit 3.12 $\mu$g/ml HIV-Antigen beschichtet worden waren, zeigten eine $E_{450}$ zwischen 1.68 und 1.74. Die Lösungen der mit 1.56 $\mu$g/ml HIV-Antigen beschichteten Wells zeigten eine $E_{450}$ zwischen 1.38 und 1.45.

Es wurden dann Wells von Microtestplatten in der zuvor beschriebenen Weise mit einer HIV-Antigenpräparation von ausschließlich 2.5 $\mu$g/ml behandelt. Die Auswertung der 16 Seren in den mit 2.5 $\mu$g/ml HIV-Antigenpräparation behandelten Well ergab bei gleichem Versuchsablauf ein mittlere $E_{450}$ von 1.56 ±0.05.

Da bei der Bestimmung von Anti-HIV eine Grenzwertfestlegung von E größer als 0.8 für Anti-HIV-negative und von E kleiner als 0.8 für Anti-HIV-positive Proben als günstig angesehen wird (der Grenzwert ist definiert als die Hälfte der mittleren Extinktion von als negativ bestätigten Proben), wurde für die Herstellung von Microtestplatten zur Bestimmung von Anti-HIV (Anti-HIV Testplatten) bei der Behandlung der Wells, die auch als Beschichtung bezeichnet wird, die Konzentration von 2.5 $\mu$g/ml HIV-Antigenpräparation gewählt.

Auch wurden bei allen im folgenden beschriebenen Bestimmungen von Anti-HIV die in den bisher beschriebenen Vorversuchen verwendeten Reagenzien, namlich das Anti-HIV-POD die TMB-Substratzubereitung und die im weiteren als Waschpuffer bezeichnete Lösung von lg/l [R]Tween 20 in PBS benutzt.

5. Bestimmung von humanen Antikörpern gegen HIV

a) 25 $\mu$l Serum oder Plasma und 100 $\mu$l Anti-HIV-POD wurden in Wells von Anti-HIV Testplatten gegeben und 1 h bei 37°C inkubiert. Der Inhalt der Wells wurde durch Absaugen entfernt und die Wells viermal mit Waschpuffer gewaschen. Es wurden in jede Well 100 $\mu$l TMB-Substratzubereitung gegeben, 30 min bei 20-22°C inkubiert und die Inkubation durch Zugabe von 100 $\mu$l 1 normale Schwefelsäure beendet. $E_{450}$ der gefärbten Lösung wurde gegen einen Leerwert von PBS gemessen.

Als Anti-HIV-positiv wurden solche Proben eingestuft, die eine $E_{450}$ von kleiner als 0.30 erzeugten, als Anti HIV-grenzwertig Proben, deren $E_{450}$ im Bereich von 0.31 bis 0.80 lag und als Anti HIV negativ Proben, die eine $E_{450}$ über 0.81 erzeugten.

Von 420 Proben, die in der Western Blot-Technik Anti-HIV positiv waren, wurden 419 Proben ebenfalls als positiv gefunden.

Von 1896 Plasma oder Serumproben gesunder Personen oder von Probanden, die andere pathologische Zustände erkennen ließen, wurde der in Tabelle 1 dargestellte Befund erhoben.

TABELLE 1

| | n | Positive oder grenzwertige Proben | Bei Wiederholung positive Proben |
|---|---|---|---|
| Normalkollektiv | 1150 | 0 | 0 |
| Proben aus Risikogruppen | 329 | 5 | 2 |
| Lupus Erythematodes | 30 | 0 | 0 |
| Seren mit Antikörpern gegen | | | |
| Leukozytäre Antigene | 29 | 0 | 0 |
| Nukleäre Antigene | 5 | 0 | 0 |
| Mitochondriale Antigene | 5 | 0 | 0 |
| HBsAg-positive Seren | 16 | 0 | 0 |
| Anti EBV positive Seren | 17 | 0 | 0 |
| Seren mit erhöhten Werten von | | | |
| Triglyceriden | 5 | 0 | 0 |
| Bilirubin | 5 | 0 | 0 |
| Hämolytische Seren | 5 | 0 | 0 |
| Proben von denselben Probanden | | | |
| Seren | 100 | 0 | 0 |
| Citrat-Plasmen | 100 | 0 | 0 |
| Heparin-Plasmen | 100 | 0 | 0 |

Die 5 Proben von Probanden aus Risikogruppen, die initial positiv oder grenzwertig reagierten, wurden zusätzlich mit der Western-Blot-Technik untersucht. Davon waren auch nur die 2 Proben positiv, die nach dem erfindungsgemäßen Verfahren als wiederholt positiv gefunden worden waren.

Die Befunde zeigen somit eine gute Übereinstimmung des erfindungsgemäßen Verfahrens mit der Western Blot-Technik.

7

b) Proben von 50 $\mu$l Vollblut wurden mit 50 $\mu$l Aqua dest., enthaltend 20 g/l [R]Triton x 100 und 0,2 mol/l Trinatriumcitrat, verdünnt und 50 $\mu$l dieses Lysates in Wells von Anti-HIV Testplatten gegeben. Nach einer Inkubation von 1 h bei 37°C erfolgt die Zugabe von 100 $\mu$l Anti-HIV-POD mit anschließender Inkubation von nochmals 1 h bei 37°C. Der Inhalt der Wells wurde durch Absaugen entfernt und die Wells fünfmal mit Waschpuffer gewaschen. Es wurden in jede Well 100 $\mu$l TMB-Substratzubereitung gegeben, 30 min. bei 20-25°C inkubiert und die Reaktion durch Zugabe von 100 $\mu$l l normaler Schwefelsäure beendet. Die Extinktion der Lösungen wurden bei 450 nm gegen einen Leerwert von PBS gemessen ($E_{450}$).

Die Bewertung der Proben erfolgt wie unter 5 a) beschrieben. Von 51 bzw. 5 Blutproben, die als Serum in der Western-Blot-Technik positiv bzw. schwach positiv waren, wurden alle Proben ebenfalls positiv gefunden.

Von 226 Blutproben, die als Serum Anti-HIV-negativ reagierten, wurden alle Proben ebenfalls als negativ gefunden. Damit wird deutlich, daß das erfindungsgemäße Verfahren gut mit der als Referenzmethode geltenden Western-Blot-Technik übereinstimmt.

c) Proben von 50 $\mu$l Serum oder Plasma oder von 50 $\mu$l Lysat von Vollblut hergestellt wie in 5 b) beschrieben wurden mit 100 $\mu$l einer Lösung von 5 $\mu$g/ml IgG-POD, hergestellt durch Verdünnen des in Beispiel 2 beschriebenen IgG-POD Konjugats von 440 $\mu$g/ml auf 5 $\mu$g/ml mit der bereits beschriebenen Lösung enthaltend foetales Kälberserum und [R]Tween 20 in PBS, in die Wells der Microtestplatten gegeben und 10 min bei 45°C inkubiert.

Anschließend wurden die Wells 5 mal mit Waschpuffer gewaschen. Dann wurde in jede Well 100 $\mu$l TMB-Substratzubereitung gegeben und 5 min bei 20-22°C inkubiert. Die Inkubation wurde durch Zugabe von 1 normaler Schwefelsäure beendet. Die Auswertung erfolgte wie in 5 a) beschrieben. Von 51 bzw. 5 Proben, die als Serum in der Western-Blot-Technik positiv bzw. schwach positiv waren, wurden alle Proben ebenfalls positiv gefunden.

Von 225 Proben, die Anti-HIV-negativ reagierten, wurden alle Proben ebenfalls negativ gefunden.

Damit wird deutlich, daß die erfindungsgemäße Variante eines schnellen Verfahrens hinsichtlich Sensitivität und Spezifität gut mit der als Referenzmethode geltenden Western-Blot-Technik übereinstimmt.

d) In die Wells von Anti-HIV-Testplatten wurden 100 $\mu$l Anti-HIV-POD gegeben und 1 h bei 37°C inkubiert, das Anti-HIV-POD abgesaugt und die Wells viermal mit Waschpuffer gewaschen. Die derart behandelten Anti-HIV-Testplatten wurden über Kieselgel bei 20-22°C getrocknet.

Anschließend wurde in die Wells 25 $\mu$l Probe und 100 $\mu$l einer Lösung von 10 g/l Rinderserumalbumin in 50 mmol/l Tris, mit HCl eingestellt auf pH 7.4, gegeben. Nach Inkubation bei 37°C während 1 h wurde der Inhalt der Wells entnommen und in Wells einer nicht beschichteten Testplatte gegeben, dort mit 100 $\mu$l TMB-Substratzubereitung versetzt, die Mischung 30 min bei 20-22°C belassen und mit 100 $\mu$l 1 normaler Schwefelsäure versetzt. $E_{450}$ wurde anschließend gegen PBS gemessen.

Die Mittelwerte $E_{450}$ von dreifacher Bestimmung pro Probe sind in Tabelle 2 dargestellt für Proben, die nach dem unter a) beschriebenen Verfahren als Anti-HIV-negativ, als Anti-HIV-grenzwertig und als Anti-HIV-positiv eingestuft worden waren.

TABELLE 2

$E_{450}$

| Anti-HIV-negative Proben | 1 | 0.140 | 0.148 | 0.136 |
| | 2 | 0.218 | 0.206 | 0.205 |
| | 3 | 0.267 | 0.279 | 0.270 |
| | 4 | 0.275 | 0.274 | 0.272 |
| Anti-HIV-grenzwertige Proben | 5 | 0.609 | 0.454 | 0.457 |
| | 6 | 0.308 | 0.316 | 0.351 |
| Anti-HIV-positive Proben | 7 | 0.769 | 0.674 | 0.773 |
| | 8 | 0.891 | 0.865 | 0.820 |
| | 9 | 1.202 | 0.953 | 0.863 |
| | 10 | 1.678 | 2.020 | 2.202 |

Tabelle 2 zeigt, daß unterschiedlich zu den Ausführungsformen a) bis c) hier die Extinktion bei Anti-HIV-negativen Proben niedrig und bei Anti-HIV-positiven Proben hoch ist. Der Grenzwert für Anti-HIV-negative Proben bei $E_{450}$ ist auch hier mit 0.30 anzusetzen.

**Patentansprüche**

1. Immunchemisches, kompetitives Verfahren zum Nachweis und zur Bestimmung von Antikörpern gegen HIV unter Verwendung einer Festphase bestehend aus einem Träger und daran gebundenen HIV Virus Proteinen, erhalten aus HIV Kulturen, und einer markierten Antikörperpräparation, wobei die markierten und unmarkierten Antikörper um die Bindungsstelle an der Festphase konkurrieren und die Festphasenproteine direkt oder über einen nichtimmunchemisch bindenden Spacer an den Träger gebunden sind, dadurch gekennzeichnet, daß die Antikörperpräparation eine Kombination von mindestens zwei Antikörperspezifitäten enthält, von denen eine gegen das Core Protein p24 und die andere mindestens gegen ein Hüllprotein aus der Gruppe gp41 oder gp120 gerichtet ist und der markierte Antikörper nicht mit Antigenen aus einer HIV-freien Wirtszelle und einem HIV-freien Medium und nicht mit einem HIV-freien humanen Gewebe reagiert.

2. Verfahren gemäß Anspruch 1 dadurch gekennzeichnet, daß die zu bestimmenden Antikörper und die markierten Antikörper gleichzeitig zur Festphase gegeben werden.

3. Verfahren gemäß Anspruch 1 dadurch gekennzeichnet, daß zunächst die markierten Antikörper zu den Festphase-gebundenen Proteinen gegeben werden und nach einer Inkubationszeit und gegebenenfalls nach Entfernen der Flüssigkeitsphase und Waschen der Festphase die Probe mit dem nachzuweisenden Antikörper zur Festphase gegeben wird.

4. Verfahren gemäß Anspruch 1 dadurch gekennzeichnet, daß zunächst die Festphase mit der Probe, die den zu bestimmenden Antikörper enthält, inkubiert wird und dann markierte Antikörper zugegeben werden.

5. Verfahren gemäß Anspruch 1 dadurch gekennzeichnet, daß die markierten Antikörper monoklonale Antikör per sind.

6. Testbesteck zum Nachweis und zur Bestimmung von Antikörpern gegen HIV in einem immunchemischen, kompetitiven Verfahren, enthaltend einen Träger und daran gebundenen HIV Virus Proteinen erhalten aus HIV Kulturen und einer markierten Antikörperpräparation, wobei die Festphasenproteine direkt oder über einen nicht-immunchemisch bindenden Spacer an den Träger gebunden sind und gegebenenfalls Reagenzien zum Nachweis der Markierung dadurch gekennzeichnet, daß Antikörperpräparation eine Kombination von mindestens zwei Antikörperspezifitäten enthält, von denen eine gegen das Core Protein p24 und die andere mindestens gegen ein Hüllprotein aus der Gruppe gp41 oder gp120 gerichtet ist und der markierte Antikörper nicht mit Antigenen aus einer HIV-freien Wirtszelle und einem HIV-freien Medium und nicht mit einem HIV-freien humanen Gewebe reagiert.

7. Testbesteck nach Anspruch 6 dadurch gekennzeichnet, daß der markierte Antikörper als ein von der Festphase getrennter Bestandteil vorliegt.

8. Testbesteck nach Anspruch 6 dadurch gekennzeichnet, daß der markierte Antikörper als ein an die Festphase immunchemisch gebundener Bestandteil vorliegt.

9. Testbesteck nach mindestens einem der Ansprüche 6 bis 8 dadurch gekennzeichnet, daß es aus einem Element besteht, in dem die Festphase und in trockener Form die für den Nachweis und für die Bestimmung erforderlichen Reagenzien ganz oder teilweise enthalten sind.

10. Verwendung eines Testbestecks nach mindestens einem der Ansprüche 6 bis 9 in einem Verfahren zum Nachweis und zur Bestimmung von Antikörpern gegen HIV.

## Claims

1. An immunochemical, competitive method for the detection and for the determination of antibodies against HIV using a solid phase comprising a support and HIV virus proteins bound thereto, obtained from HIV cultures, and a labeled antibody preparation, the labeled and unlabeled antibodies competing for the binding site on the solid phase and the solid phase proteins being bound to the support directly or via a non-immunochemically binding spacer, wherein the antibody preparation contains a combination of at least two antibody specificities, of which one is directed against the core protein p24 and the other at least against a coat protein of the group gp41 or gp120 and the labeled antibody does not react with antigens from an HIV-free host cell and an HIV-free medium and does not react with an HIV-free human tissue.

2. The method as claimed in claim 1, wherein the antibodies to be determined and the labeled antibodies are added to the solid phase simultaneously.

3. The method as claimed in claim 1, wherein the labeled antibodies are first added to the solid phase-bound proteins and after an incubation period and, if desired, after removing the liquid phase and washing the solid phase, the sample containing the antibody to be detected is added to the solid phase.

4. The method as claimed in claim 1, wherein first the solid phase is incubated with the sample which contains the antibody to be determined, and labeled antibodies are then added.

5. The method as claimed in claim 1, wherein the labeled antibodies are monoclonal antibodies.

6. A test kit for the detection and for the determination of antibodies against HIV in an immunochemical, competitive method, containing a support and HIV virus proteins bound thereto and obtained from HIV cultures and a labeled antibody preparation, the solid phase proteins being bound to the support directly or via a non-immunochemically binding spacer, and optionally reagents for the detection of the labeling, wherein the antibody preparation contains a combination of at least two antibody specificities, of which one is directed against the core protein p24 and the other at least against a coat protein from the group gp41 or gp120 and the labeled antibody does not react with antigens from an HIV-free host cell and an HIV-free medium and does not react with an HIV-free human tissue.

7. A test kit as claimed in claim 6, wherein the labeled antibody is present as a constituent separate from the solid phase.

# EP 0 265 851 B1

8. A test kit as claimed in claim 6, wherein the labeled antibody is present as a constituent immunochemically bound to the solid phase.

9. A test kit according to at least one of claims 6 to 8, wherein it comprises an element in which the solid phase and the reagents necessary for the detection and for the determination are completely or partially contained in dry form.

10. The use of a test kit according to at least one of claims 6 to 9 in a method for the detection and for the determination of antibodies against HIV.

## Revendications

1. Procédé immunochimique compétitif pour détecter et pour doser des anticorps dirigés contre le VIH, avec utilisation d'une phase solide constituée d'un substrat et de protéines du VIH liées à celui-ci, obtenues à partir de cultures de VIH, et d'une préparation d'anticorps marqués, les anticorps marqués et non marqués entrant en concurrence pour les sites de liaison à la phase solide et les protéines de la phase solide étant liées au support de façon directe ou par l'intermédiaire d'un segment intercalaire se liant par voie non immunochimique, caractérisé en ce que la préparation d'anticorps contient une combinaison d'au moins deux catégories spécifiques d'anticorps dont l'une est dirigée contre la protéine-noyau p24 et l'autre est dirigée contre au moins une protéine-enveloppe des groupes gp41 ou gp120, et l'anticorps marqué ne réagit pas avec des antigènes provenant d'une cellule-hôte dépourvue de VIH et d'un milieu dépourvu de VIH, et il ne réagit pas avec un tissu humain dépourvu de VIH.

2. Procédé selon la revendication 1, caractérisé en ce que l'on ajoute simultanément les anticorps à déterminer et les anticorps marqués à la phase solide.

3. Procédé selon la revendication 1, caractérisé en ce que l'on ajoute d'abord les anticorps marqués aux protéines liées à la phase solide et, après une période d'incubation et, éventuellement, après élimination de la phase liquide et lavage de la phase solide, on ajoute à la phase solide l'échantillon contenant l'anticorps à détecter.

4. Procédé selon la revendication 1, caractérisé en ce que l'on incube d'abord la phase solide avec l'échantillon contenant l'anticorps à déterminer, et on ajoute ensuite des anticorps marqués.

5. Procédé selon la revendication 1, caractérisé en ce que les anticorps marqués sont des anticorps monoclonaux.

6. Trousse de test pour détecter et pour doser des anticorps dirigés contre le VIH dans un procédé immunochimique compétitif, contenant un substrat et des protéines du VIH liées à celui-ci, obtenues à partir de cultures de VIH, ainsi qu'une préparation d'anticorps marqués, les protéines de la phase solide étant liées au support de façon directe ou par l'intermédiaire d'un segment intercalaire se liant par voie non immunochimique, et, éventuellement, des réactifs pour détecter le marquage, caractérisée en ce que la préparation d'anticorps contient une combinaison d'au moins deux catégories spécifiques d'anticorps dont l'une est dirigée contre la protéine-noyau p24 et l'autre est dirigée contre au moins une protéine-enveloppe des groupe gp41 ou gp120, et l'anticorps marqué ne réagit pas avec des antigènes provenant d'une cellule-hôte dépourvue de VIH et d'un milieu dépourvu de VIH, et il ne réagit pas avec un tissu humain dépourvu de VIH.

7. Trousse de test selon la revendication 6, caractérisée en ce que l'anticorps marqué est présent sous la forme d'un des composants distincts de la phase solide.

8. Trousse de test selon la revendication 6, caractérisée en ce que l'anticorps marqué est présent sous la forme d'un composant lié par voie immunochimique à la phase solide.

9. Trousse de test selon au moins une des revendications 6 à 8, caractérisée en ce qu'elle est constituée d'un élément qui contient, en partie ou en totalité, la phase solide et, sous forme anhydre, les réactifs nécessaires à la détection et au dosage.

**10.** Utilisation d'une trousse de test selon au moins une des revendications 6 à 9 dans un procédé pour détecter et pour doser des anticorps dirigés contre le VIH.